# EUROPÄISCHE PATENTSCHRIFT

(11) **EP 0 306 473 B1**
(45) Veröffentlichungstag und Bekanntmachung des Hinweises auf die Patenterteilung: **18.11.1993**
(21) Anmeldenummer: 88890212.9
(22) Anmeldetag: 19.08.1988
(51) Int. Cl.: A61K 39/385, A61K 47/00, A61K 37/00, C12N 11/06, C12N 11/08, A61K 37/02

(54) **Pharmazeutische Struktur mit an Proteinträger gebundenen Wirkstoffen**
Pharmaceutical structure with protein-supported agents
Structure pharmaceutique avec agents liés à des supports protéiniques

(30) Priorität: 21.08.1987 DE 3727987
(43) Veröffentlichungstag der Anmeldung: 08.03.1989
(73) Patentinhaber: Sleytr, Uwe B., Dipl.-Ing., Dr., A-1170 Wien (AT)
(72) Erfinder: Sleytr, Uwe B., Prof. Dipl.-Ing. Dr., A-1170 Wien (AT); Mundt, Wolfgang, Dipl.-Ing. Dr., A-1080 Wien (AT); Messner, Paul, Dipl.-Ing. Dr., A-1140 Wien (AT)
(74) Vertreter: Itze, Peter, Dipl.-Ing.

(56) Entgegenhaltungen:
- EP-A- 0 037 388
- EP-A- 0 060 422
- EP-A- 0 118 831
- EP-A- 0 180 564
- EP-A- 0 191 536
- EP-A- 0 198 388
- AT-B- 284 344
- AT-B- 373 278
- CH-A- 632 789
- US-A- 4 411 832

## Beschreibung

Die Erfindung bezieht sich auf eine pharmazeutische Struktur, in welcher Haptene und/oder immunogene bzw. immunstimulierende Substanzen an einen Proteinträger gebunden sind.

Es wurde bereits versucht, Haptene und/oder immunogene bzw. immunstimulierende Substanzen an Proteine, wie z.B. Serumalbumin, zu binden und dadurch deren Immunogenität bzw. Wirkung zu erhöhen. Bei diesen bekannten Ausbildungen liegen die Proteinmoleküle ungeordnet in Lösung oder als unstrukturierte Aggregate vor. Die Haptenbindungsstellen oder die Bindungsstellen der immunogenen bzw. immunstimulierenden Substanzen können sich dabei nach ihrer Natur, Art, Zahl und Lage in den Proteinmolekülen erheblich unterscheiden, wodurch keine gut definierte Bindung der Haptene und/oder immunogenen bzw. immunstimulierenden Substanzen an den Proteinmolekülen möglich ist.

AT-PS 373278 offenbart die Immobilisierung von Mikrobenzellen mit einer Glucoseisomeraseaktivität durch Bindung an Blutserum bzw. Blutplasma. Auf diese Art wird ein dreidimensionales Gebilde geschaffen, das eine amorphe ungeordnete Struktur aufweist. Bei diesem bekannten Verfahren werden die Mikrobenzellen mit Glutaraldehyd in Anwesenheit von Blutserum oder Blutplasma als Eiweißträger polymerisiert. In diesem Blutplasma oder Blutserum ist eine Mischung aus fibrillären oder globulären Proteinen vorhanden, die ungeordnet in einer homogenen Phase vorliegen und durch Glutaraldehyd mit den Bakterienzellen verbunden werden, sodaß die Bakterienzellen, die alleine nur bedingt polymerisierbar sind, zu einem festen Gebilde vereinigt werden. Dazu dienen die fibrillären Globuline als gegenseitige Anker zwischen den entsprechenden Mikrobenzellen. Es handelt sich damit um ein dreidimensionales Gebilde, wobei die aktiven Zellen aufgrund einer zufälligen Vernetzung in einer statistischen Verteilung vorliegen.

Die Schweizer Patentschrift 632789 offenbart die Fixierung von Enzymen oder Enzympräparaten auf einem quellfähigen, gehärteten (denaturierten) Protein, wobei die Enzyme mittels Glutaraldehyd in Gegenwart einer enzymfällend wirkenden Substanz gebunden wird. Dadurch entstehen Präparate, bei denen das Enzym an bestimmten Stellen auf der Außenseite und im Inneren des hochmolekular-porösen Trägerproteins mit vorgegebener Struktur gleichsam "angehängt" ist. Als Ausgangsmaterial werden dabei wasserlösliche Proteine verschiedener Art, z.B. Gelatine, Eiklar, Albumin, Sojaprotein und ähnliches verwendet. Wesentlich ist, daß diese Proteine durch Härtung wasserunlöslich gemacht und gegebenenfalls durch sonstige Behandlung so modifiziert werden können, daß sie im Temperaturbereich zwischen 0 und 100C eine im wesentlichen gleichbleibende Wasseraufnahmefähigkeit vom zwei- bis achtfachen ihres Trockengewichtes erhalten. Derartig vernetzte Proteine bilden aber ungeordnete Matrices, u.zw. bildet z.B. Gelatine ein ungeordnetes dreidimensionales Netzwerk fadenförmiger Moleküle, welches dann mit Aldehyden stabilisiert und gehärtet wird. Auch hier trifft zu, daß nur eine statistische Verteilung der immobilisierten Enzyme erfolgt.

Die EP-A2-0-180 564 betrifft die Bildung von Trägerstrukturen, die sogenannten "Iscoms", welche einen Komplex zwischen einem Glycosid (Adjuvans), Lipiden und einem antigenen hydrophoben Peptid oder Protein darstellen. In einem ersten Reaktionsschritt muß das hydrophobe Antigen durch nicht-kovalente Bindung, d.h. hydrophobe Wechselwirkung, an die Glycosid (Adjuvans)-Lipid-Matrix gebunden werden. Der entstehende dreidimensionale Komplex hat eine käfigartige Struktur. Es gibt im ersten Reaktionsschritt somit keine regelmäßige und genau geordnete Immobilisierungsmöglichkeit von antigenen Molekülen.

EP-A2-0 118 831 beschreibt die Bindung von Antigenen an einen Trägerkörper, bei welchem Proteine und lipidfreie Polysaccharide von Gram-negativen Bakterien aneinander gebunden werden. Als Trägermaterial wird detoxifiziertes Protein verwendet.

Das in EP-A2-0118831 beschriebene detoxifizierte Protein von Gram-negativen Bakterien bildet keine zweidimensionalen Kristallgitter. Daraus resultiert ein vollkommen anderes Präparationsverfahren des Proteinträgers. Das für die Kopplung selektiv oxidierte detoxifizierte Polysaccharid liegt unstrukturiert in homogener Lösung vor.

In EP-A1- 0 191 536 ist die Bildung eines synthetischen Immunogens beschrieben, bei welchem die immunogene Struktur durch die Ausbildung von Micellen beschrieben ist. Micellen sind dreidimensionale kugelige Strukturen, bei welchen die funktionellen Gruppen je nach ihrer Endgruppe, hydrophil oder hydrophob entsprechend ausgerichtet sind. An diesen dreidimensionalen Strukturen sind dann immunogene Wirkgruppen gekoppelt.

In US-A- 4 411 832 ist eine Trägermatrix aus einem Polysaccharid, nämlich Agarose, beschrieben. Agarose besteht aus Polysacchariden mit einer vorgegebenen Größenverteilung; wobei die einzelnen Moleküle kein zweidimensionales Gitter ausbilden können.

Der Erfindung liegt die Aufgabe zugrunde, eine pharmazeutische Struktur der eingangs genannten Art zu schaffen, bei welcher eine genau definierte Zahl von Bindungsstellen vorhanden ist, um eine genau definierte Menge von Haptenen und/oder immunogenen bzw. immunstimulierenden Substanzen in genau definierter Weise an den Träger binden zu können.

Erfindungsgemäß wird diese Aufgabe dadurch gelöst, daß der Proteinträger durch kristallin oder parakristallin aneinanderliegende, gegebenenfalls miteinander kovalent vemetzte, Proteinmoleküle oder proteinhältige Moleküle einer S-Schicht gebildet ist. Durch die kristalline oder parakristalline Struktur der S-Schicht weisen die Proteinmoleküle immer eine genau bestimmte räumliche Ausrichtung zueinander auf; dadurch sind sowohl die Art der Bindung, wie auch die Anzahl und die räumliche Orientierung der gebundenen Haptene und/oder immunogenen bzw. immunstimulierenden Substanzen und die Entfernung der Haptenbindungsstellen oder der Bindungsstellen der immunogenen bzw. immunstimulierenden Substanzen voneinander immer genau definiert.

Weiters ist es möglich, die Proteinträger so zu wählen, daß sie auf Grund ihrer Form, ihrer Größe, ihrer Anordnung und Oberflächeneigenschaften bevorzugt phagozytiert werden. Dadurch wird die Aufnahme der Haptene und/oder immunogenen bzw. immunstimulierenden Substanzen durch eine phagozytierende Zelle, z.B. einen Makrophagen, wesentlich begünstigt. Im Gefolge wird eine wirkungsvollere Immunantwort erzielt.

Vorteilhafterweise können die kristallin oder parakristallin aneinanderliegenden proteinhältigen Moleküle Glycoproteine sein, wodurch die Struktur des Trägers der Zelloberflächenstruktur bzw. der Form von Bakterien noch besser angenähert werden kann. Dabei können die kristallin aneinanderliegenden Moleküle aus einer oder mehreren Mikroorganismenzellwandschichten stammen. Dadurch werden die Glycoproteine auf besonders einfache Weise erhalten. Es können dabei an dem kristallinen oder parakristallinen Proteinträger zusätzlich weitere Zellwandbestandteile anhaften. Bei Auswahl der richtigen Mikroorganismen können auch Fragmente von Mikroorganismenzellwänden mit den Haptenen und/oder immunogenen bzw. immunstimulierenden Substanzen beladen sein. Es eignen sich dazu insbesondere jene Mikroorganismen, bei welchen außer den die kristalline Oberflächenschicht bildenden Proteinmolekülen auch darunterliegende rigide Schichten, z.B. Peptidoglycan- oder Pseudomureinschichten, vorhanden sind.

Es können dabei die Haptene und/oder immunogenen bzw. immunstimulierenden Substanzen an den Proteinanteil des Trägers gebunden sein. Für andere Haptene kann es von Vorteil sein, wenn sie gegebenenfalls an den Kohlenhydratanteil der Glycoproteine gebunden sind. Es hängt also sowohl von den Haptenen und/oder immunogenen bzw. immunstimulierenden Substanzen als auch von der Anwendung der pharmazeutischen Struktur ab, welche der beiden Bindungen gewählt wird, wobei unter gewissen Umständen auch Mischformen der beiden vorteilhaft sein können.

Weiters können die Haptene und/oder immunogenen bzw. immunstimulierenden Substanzen an dem jeweiligen Trägermolekül über Zwischenmoleküle, wie z.B. homo- oder heterobifunktionelle quervernetzende Agentien (Crosslinker) oder Peptidketten (z.B. Polylysin), gebunden sein. Die Einführung solcher sogenannter "spacer" bzw. aktiver Zwischenmoleküle hat den Vorteil, daß dadurch die Abspaltung der Haptene und/oder immunogenen bzw. immunstimulierenden Substanzen und damit die Art der Bruchstücke die durch den enzymatischen Abbau in den Endosomen (Lysosomen) der Makrophagen entstehen genauer kontrolliert werden kann. Es kann mit diesen Zwischenmolekülen eine "Sollbruchstelle" geschaffen werden, an welcher die abbauenden Enzyme bevorzugt angreifen.

Schließlich können unterschiedliche Träger und/oder mit unterschiedlichen Haptenen und/oder immunogenen bzw. immunstimulierenden Substanzen beladene Träger vereinigt sein. Dadurch wird erreicht, daß unterschiedliche Funktionen in der pharmazeutischen Struktur erzielt werden, nämlich daß Trägermoleküle, die stark hydrophob sind, gemeinsam mit den mit Haptenen und/oder immunogenen bzw. immunstimulierenden Substanzen, beladenen Trägern wirksam werden, was eine verbesserte Aufnahme der pharmazeutischen Struktur durch die phagozytierenden Zellen zur Folge hat. Weiters können auch mit unterschiedlichen Haptenen und/oder immunogenen bzw. immunstimulierenden Substanzen beladene Träger vorgesehen sein, wodurch die Wirkcharakteristik der pharmazeutischen Struktur genau gesteuert werden kann.

Bei einem vorteilhaften Verfahren zur Herstellung der erfindungsgemäßen pharmazeutischen Struktur können die die Haptene und/oder immunogenen bzw. immunstimulierenden Substanzen bindenden Gruppen der Proteinmoleküle oder proteinhältigen Moleküle vor dem Zusatz der Haptene aktiviert werden. Dadurch wird eine zuverlässig genaue und reproduzierbar stabile Bindung der Haptene und/oder immunogenen bzw. immunstimulierenden Substanzen an die entsprechenden Gruppen erzielt.

Zur Bindung der Haptene und/oder immunogenen bzw. immunstimulierenden Substanzen an die Kohlenhydratanteile können Bindungsstellen an den Kohlenhydratanteilen der Glycoproteine durch Oxydation, beispielsweise durch Perjodat, geschaffen werden. Es kann die Bildung der Bindungsstellen an den Proteinmolekülen durch das die Proteinmoleküle kovalent vernetzende Agens, z.B. Glutaraldehyd, vorgenommen werden, wodurch das Vernetzen der Proteinmoleküle in einem Arbeitsgang gleichzeitig aktive Gruppen für die Bindung der Haptene und/oder immunogenen bzw. immunstimulierenden Substanzen erzeugt. Es kann die Bildung der Bindungsstellen auch durch Einführung aktiver Gruppen vorgenommen werden, wodurch eine noch genauere Steuerung der Zahl und der Art der Bindungen erreicht wird. Für eine besonders stabile Bindung können die Haptene und/oder immunogenen bzw. immunstimulierenden Substanzen an die Carboxylgruppen des Proteinträgers amidartig gebunden werden. Für gewisse aktive Bindungsstellen kann die Bindung der Haptene und/oder immunogenen bzw. immunstimulierenden Substanzen in Form von Schiff'schen Basen erfolgen, wobei gegebenenfalls die Schiff'schen Basen zu sekundären Aminen reduziert werden können.

Weiters können spezielle Bindungen, bzw. speziell aufspaltbare Bindungen dadurch erreicht werden, daß an beiden Enden aktivierte Bindungsstellen aufweisende Zwischenmoleküle, wie z.B. homo- oder heterobifunktionelle quervernetzende Agentien (Crosslinker) oder Peptidketten (z.B. Polylysin), mit einem Ende an den Träger gebunden werden, wonach dann das Hapten und/oder die immunogene bzw. immunstimulierende Substanz an das andere Ende des Zwischenmoleküls gebunden wird. Auf diese Weise werden dann sogenannte "spacer" in die Struktur eingeführt.

Schließlich können unterschiedliche Träger und/oder mit unterschiedlichen Haptenen und/oder immunogenen bzw. immunstimulierenden Substanzen beladene Träger auf eine Hilfsschicht aufgebracht werden, welche nach Vernetzen der Träger gegebenenfalls entfernt wird. Damit ist die Möglichkeit gegeben, pharmazeutische Strukturen zu schaffen, welche unterschiedliche Trägermoleküle, die sich beispielsweise durch die kristalline Struktur od.dgl. voneinander unterscheiden, in einem pharmazeutischen Präparat zu vereinigen. Es können aber auch solche Träger, die unterschiedliche Haptene und/oder immunogene bzw. immunstimulierende Substanzen tragen, gleichfalls zu einer pharmazeutischen Struktur vereinigt werden.

Mit den gleichen Vorteilen können Bindungsstellen am Hapten und/oder an der immunogenen bzw. immunstimulierenden Substanz aktiviert und die Haptene und/oder die immunogenen bzw. immunstimulierenden Substanzen über diese aktivierten Bindungsstellen an den Proteinträger oder Glykoproteinträger gebunden werden, was ebenfalls besonders stabile Bindungen ergibt. Die Erfindung wird nachstehend noch an Hand von Beispielen näher erläutert.

### Beispiel 1

### A Herstellung des Trägers

Zellen von Clostridium thermohydrosulfuricum L111-69 (2.5g) werden in 50mM Tris-HCl-Puffer, pH 7,2 suspendiert und kurz, etwa 1 min mit Ultraschall behandelt. Nach Zusatz von 12,5 ml 2%iger Triton X-100 Lösung wird die Suspension 15 min bei 50°C inkubiert. Durch diese Behandlung wird das Cytoplasma und die Cytoplasmamembran der Zellen desintegriert, wogegen die kristalline oder parakristalline proteinhältige Zellwandschicht (im folgenden "S-Schicht" genannt) und die darunterliegende Peptidoglycanschicht als Fragmente erhalten bleiben. Anschließend wird bei 20.000g abzentrifugiert und zur Entfernung des Detergens dreimal mit Wasser gewaschen. Danach wird das Pellet in 5mM Magnesiumchloridlösung (25ml) suspendiert und zur Entfernung von Plasmaresten und Nucleinsäuren 125 µg DNAse sowie 500 µg RNAse zugesetzt und 15 min bei 37°C gerührt. Anschließend wird dreimal mit Wasser gewaschen, wobei bei 20.000g zentrifugiert wird. Das Pellet wird in 20 ml 0,1M Kakodylatpuffer pH 7,2 suspendiert und mit einer 50%igen Lösung von Glutaraldehyd in Wasser bei 4°C bis zu einer Endkonzentration von 0.5% versetzt. Dann wird bei 4°C einige Minuten stark gerührt, zentrifugiert und mit Wasser gewaschen. Das Pellet wird in 25 ml Wasser suspendiert und danach Tris-hydroxymethylaminomethan ("Tris") zugesetzt. Nach 10min Stehen bei Raumtemperatur wird erneut abzentrifugiert (20.000g) und gewaschen.

Unter gewissen Umständen kann die Behandlung der Zellen mit Ultraschall entfallen, u.zw. dann, wenn die Zellform der Mikroorganismen erhalten und nur das Plasma aus der Zelle entfernt werden soll.

Wie angeführt, bleibt bei der vorstehend beschriebenen Verfahrensweise an der proteinhältigen Zellwandschicht auch die darunterliegende Peptidoglycanschicht erhalten, wobei es bei zahlreichen Organismen zu der Ausbildung einer zusätzlichen S-Schicht kommen kann. Dies führt dazu, daß die Fragmente bzw. die leeren Mikroorganismenhüllen, die nur mehr aus S-Schicht und Peptidoglycanschicht bestehen, an der Innenseite der Peptidoglycanschicht nunmehr gleichfalls S-Schichten aufweisen, die dann mit Haptenen und/oder immunogenen bzw. immunstimulierenden Substanzen oder sonstigen Wirkstoffen beladen werden können. Ist in solchen Fällen das Peptidoglycan störend, so kann es mit Hilfe eines Peptidoglycan abbauenden Enzyms, z.B. Lysozym, entfernt werden. Dazu wird das gemäß Punkt A hergestellte Pellet mit einer Lysozymlösung (0,5 mg Lysozym per ml einer 50mM Tris-HCl-Pufferlösung, pH 7,2) eine Stunde bei 36°C behandelt. Es werden dabei auf 0,5g Naßpellet 10ml Lysozymlösung zugesetzt. Die so hergestellten S-Schicht-Fragmente bestehen je nach Mikroorganismus aus einer einfachen oder einer doppelten S-Schicht, wobei bei Ultraschallbehandlung der Zellen offene Fragmente vorhanden sind, wogegen bei Zellen, die nicht mit Ultraschall behandelt wurden, die Zellform bzw. die kristalline oder parakristalline S-Schicht unzerstört erhalten bleibt.

### B Bildung von Bindungsstellen

Das gemäß A hergestellte Pellet wird in 5 ml Wasser suspendiert, wonach 5 ml einer 0,1M Lösung von Natriumperjodat zugegeben werden. Diese Suspension wird dann 24 Std bei 4°C unter Lichtausschluß stehen gelassen. Danach wird zentrifugiert und zur Entfernung der jodhaltigen Salze mit 10mM Natriumchloridlösung gewaschen.

### C Bindung von Eiweißkörpern an die so erhaltenen S-Schichten

Das gemäß B erhaltene Pellet (etwa 0,2g) wird in 1 ml Wasser suspendiert und mit 1 ml einer Lösung von 50 mg Rinderserumalbumin pro 10 ml Wasser versetzt. Diese Lösung läßt man nun 60 min bei Raumtemperatur stehen und zentrifugiert dann ab.

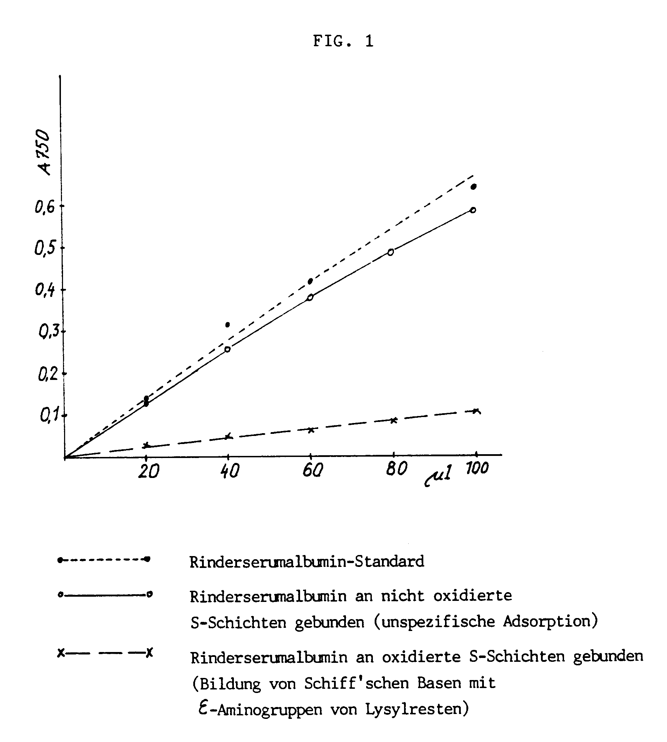

Zur Bestimmung der Menge des an den Träger gebundenen Albumins wird die Extinktion bei 750 nm im Vergleich zu einer Präparation gemessen, bei der anstelle der Oxidation mit Perjodat mit Wasser inkubiert wurde. Das Ergebnis dieser Messung ist in Fig. 1 wiedergegeben, aus welcher man deutlich erkennen kann, daß eine signifikant höhere Bindung an die mit Perjodat oxidierten S-Schichten erfolgt.

### Beispiel 2

### A Herstellung des Trägers

Zellen von Bacillus stearothermophilus PV 72 (2.5 g) werden in 50mM Tris-HCl-Puffer, pH 7,2 suspendiert und etwa 1 min mit Ultraschall behandelt. Nach Zusatz von 2% Triton X-100 (12,5 ml) wird die Suspension 15 min bei 50°C inkubiert. Durch diese Behandlung wird das Cytoplasma der Zellen desintegriert während die S-Schicht und die darunterliegende Peptidoglycanschicht erhalten bleibt. Es entstehen solcherart Fragmente die weitgehend der ursprünglichen Form der Bakterienzelle entsprechen (sogenannte "ghosts"). Anschließend wird bei 20.000g zentrifugiert und zur Entfernung des Detergens drei Mal mit Wasser gewaschen. Dann wird das Pellet in 5 mM Magnesiumchloridlösung (25ml) suspendiert, 125 µg DNAse und 500 µg RNAse zugesetzt und 15 min bei 37°C gerührt. Anschließend wird drei Mal mit Wasser gewaschen, wobei dazwischen bei 20.000g zentrifugiert wurde. Das Pellet wird in 0,1 M Kakodylatpuffer (pH 7,2) suspendiert und mit einer 50%-igen Lösung von Glutaraldehyd in Wasser bei 4°C bis zu einer Endkonzentration von 0,5% versetzt.

Die Suspension wird dann bei 4°C einige Minuten stark gerührt, zentrifugiert und mit Wasser gewaschen. Da durch diese Vorgangsweise die Glutaraldehydreste, welche mit nur einer Aldehydfunktion gebunden sind, als freie Bindungsstellen dienen, erübrigt sich die Schaffung von speziellen Bindungsstellen, wie dies gemäß Beispiel 1 B durch Oxidation bewirkt wurde.

### B Bindung von Eiweißkörpern an die so erhaltenen S-Schichten.

Die wie vorstehend beschrieben erhaltenen S-Schichten werden wie in Beispiel 1 C mit Rinderserumalbumin versetzt und die Menge an gebundenen Eiweißkörpern wie dort beschrieben bestimmt.

### Beispiel 3

### A Herstellung des Trägers

Zellwände von Clostridium thermohydrosulfuricum L111-69 werden zur Stabilisierung der äußersten Zellwandschicht (S-Schicht) mit Glutaraldehyd (0,5% in 0,1M Natrium-Kakodylat-Puffer, pH 7,2) 20 min bei 20°C behandelt. Die Reaktion wird durch Zugabe eines Überschusses von Ethanolamin gestoppt. Die Zellwandfragmente können während der Vernetzungsreaktion entweder suspendiert vorliegen oder auf eine poröse Struktur aufgebracht sein (S-Schicht-Ultrafiltrationsmembranen). Zur Entfernung des Reaktionsgemisches werden die Zellwandfragmente mit destilliertem Wasser gewaschen.

### B Schaffung von Bindungsstellen für SH-Gruppen-haltige Liganden

Danach werden 0,2 g des Pellets in 30ml dest. Wasser suspendiert und zur Aktivierung der exponierten Carboxylgruppen der S-Schicht 60 mg 1-Ethyl-3.3'(dimethylaminopropyl)carbodiimid (EDC) unter Einhaltung eines pH-Wertes von 4.75 zugefügt. Dann wird ein Überschuß von Hexamethylendiamin (0,5g) zugesetzt und der pH-Wert während der Reaktionszeit von 60 Minuten auf 8,0 konstant gehalten. Anschließend wird die Reaktion durch Zugabe von Essigsäure gestoppt, die Suspension bei 20.000g zentrifugiert und das Pellet dreimal mit dest. Wasser gewaschen. 100 mg des feuchten Pellets werden in 9 ml Phosphatpuffer (50mM, pH 7,0) suspendiert und 1 ml einer Lösung von meta-Maleimidobenzoyl N-Hydroxisuccinimidester (MBS) (50mg/ml Tetrahydrofuran) zudosiert und 30 Minuten bei 20°C inkubiert.

### C Bindung von SH-Gruppen-haltigen Eiweißkörpern an die so erhaltenen S-Schichten

Nach Zentrifugieren bei 20.000g wird das Pellet in 50mM Phosphatpuffer (pH 7,0) resuspendiert, 20 mg β-Galactosidase hinzugefügt und 2 Stunden bei 20°C inkubiert. Nach Zentrifugation bei 20.000g und wiederholtem Waschen mit Phosphatpuffer wird die Aktivität der kovalent an die Proteinmatrix gebundenen β-Galactosidase bestimmt.

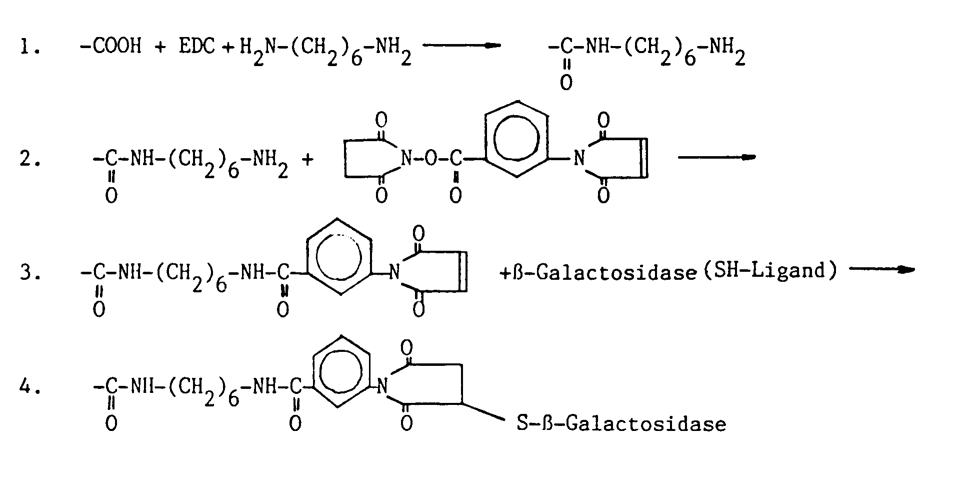

### Beispiel 4

Für die Bindung von Invertase werden die vicinalen OH-Gruppen des Kohlenhydratanteils, der kovalent an das S-Schicht-Protein gebunden ist, genutzt.

Zellwandfragmente werden wie in Beispiel 3 unter A beschrieben, zur Stabilisierung der äußersten Zellgrenzfläche mit Glutaraldehyd versetzt.

### B Schaffung von Bindungsstellen

100 mg der Zellwandfragmente werden in wasserfreiem Tetrahydrofuran (THF) suspendiert, 10 min bei 20°C inkubiert, bei 20.000g zentrifugiert und anschließend in 10ml Bromcyan-Lösung (2,5% in Tetrahydrofuran) resuspendiert. Nach 2 Stunden Inkubationszeit werden die Zellwandfragmente durch zentrifugation bei 20.000g abgetrennt und mit THF zur Entfernung von restlichem Reagens gewaschen.

### C Bindung von Eiweißkörpern an die so behandelte S-Schicht

Das Pellet wird in 10ml 50mM Phosphatpuffer (pH 8,0), der 20 mg Invertase enthält, resuspendiert und 18 Stunden bei 4°C inkubiert. Nach Zentrifugieren bei 20.000g wird das Pellet zwei Mal mit Phosphatpuffer gewaschen und die enzymatische Aktivität der an die Proteinmatrix gebundenen Invertase bestimmt.

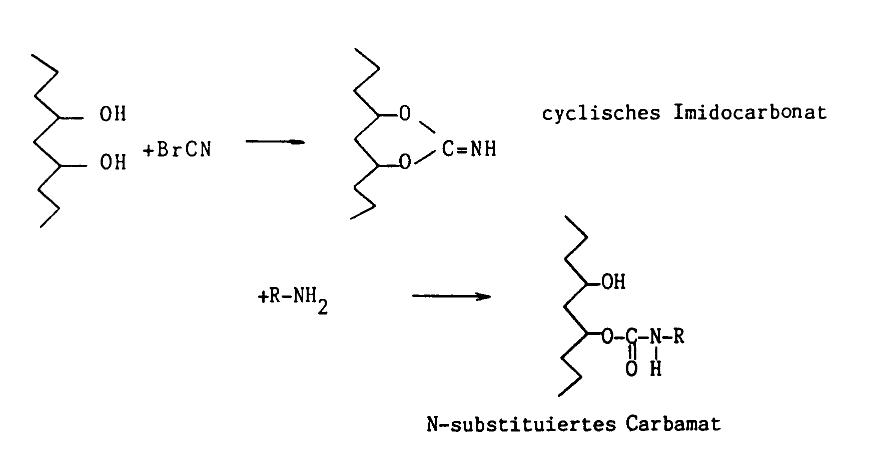

### Beispiel 5

Zellwandfragmente von Clostridium thermohydrosulfuricum L111-69 werden wie in Beispiels 3 unter A beschrieben mit Glutaraldehyd fixiert.

### B Schaffung von Bindungsstellen

0,1 g Zellwandfragmente werden in 20 ml wasserfreiem Dimethylformamid (DMF) suspendiert, und danach 60 mg EDC und 0,5 g N-Hydroxisuccinimid zugefügt. Nach einer Inkubationszeit von 1 Stunde wird die Suspension bei 20.000g zentrifugiert und zweimal mit DMF gewaschen.

### C Bindung von Eiweißkörpern an die so erhaltene S-Schicht

Das erhaltene Pellet wird in 0,1M Natriumhydrogencarbonat (pH 8,8), worin 20 mg Dextranase gelöst ist, suspendiert und der Reaktionsansatz 18 Stunden bei 4°C inkubiert. Die mit Dextranase beladenen Zellwandfragmente werden durch Zentrifugieren bei 20.000g gewonnen und zwei Mal mit dest. Wasser gewaschen. Die Dextranase-Aktivität des Pellets wird dann bestimmt.

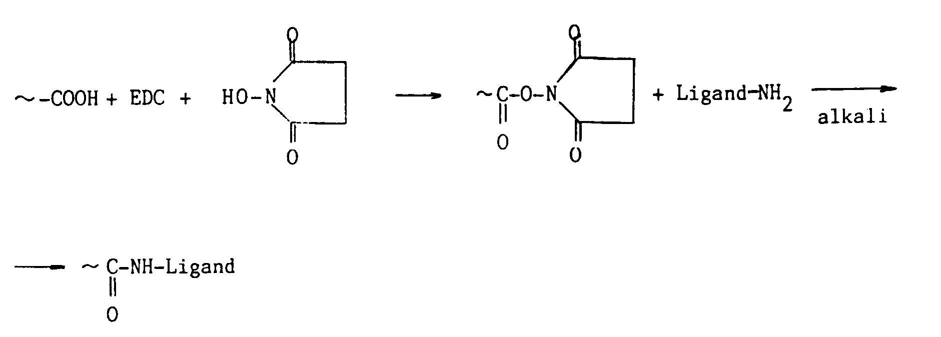

### Beispiel 6

### Koppelung eines synthetischen Kohlenhydrat-Antigens an oxidierte S-Schichten

### A Herstellung des Trägers und

### B Schaffung von Bindungsstellen

Die Herstellung von oxidierten Glycoprotein-S-Schichten wurde wie in Beispiel 1 und A und B beschrieben durchgeführt.

### C Bindung des Kohlenhydrat-Antigens an den Träger

Es wird das Polyaldehyd-Produkt mit dem 3-(2-aminoethyl)thiopropylglycosid eines Disaccharids unter Bildung einer Schiff'schen Base inkubiert. Dieser Schritt kann auch mit jedem anderen an ein Amminogruppen enthaltendes Aglycon gebundenen Saccharid ausgeführt werden.

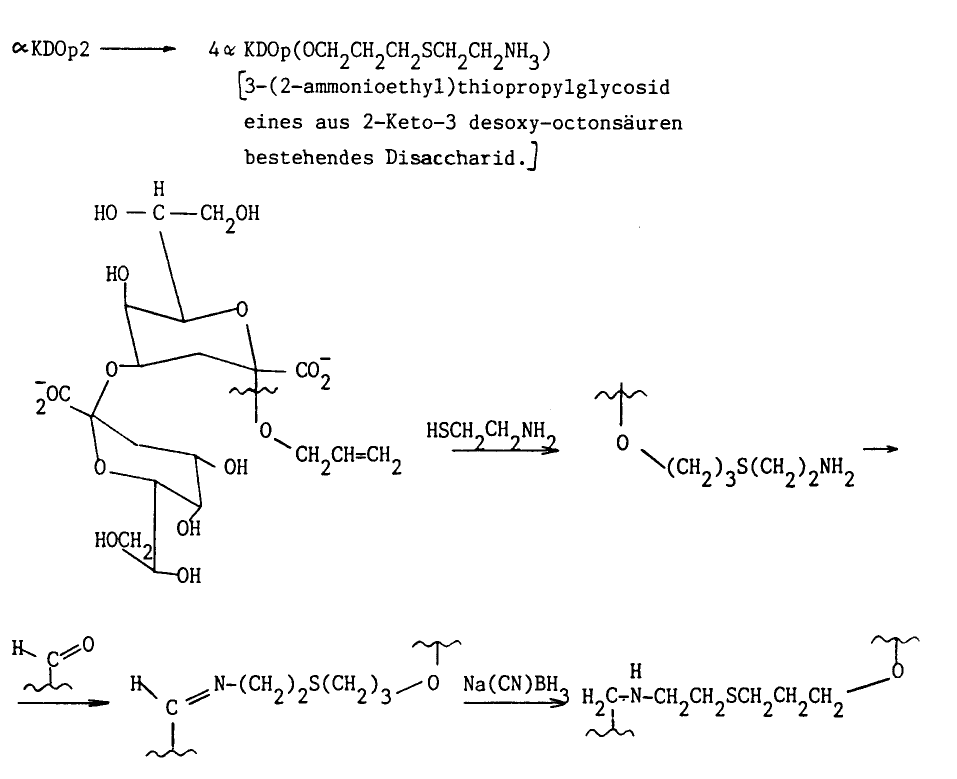

### Allgemeine Vorschrift zur Herstellung von 3-(2-Aminoethylthio)propylglycosiden aus Allylglycosiden

Eine Lösung des Allylglycosids (5mM) in 10ml einer Lösung von Cysteaminhydrochlorid (15 milliäquivalent Thiolgruppen) wird 1,5 Stunden bei Raumtemperatur stehen gelassen. Diese Reaktionsdauer kann unterschiedlich sein. Das Reaktionsgemisch wird anschließend über eine Kationen-Austauschsäule (z.B. Rexyn 101, Ammoniumform, 200-400mesh), getrennt. Man wäscht mit Wasser, 0,5M Ammoniak, und 1,0M Ammoniak. Unreagiertes Allylglycosid erscheint im wässerigen Eluat, und das 3-(2-Aminoethylthio)propylglycosid bei 1,0M Ammoniak. Die die jeweiligen Produkte enthaltenden Fraktionen werden in der Folge eingedampft.

Die durch die Bindung des 3-(2-Aminoethylthio)propylglycosids erhaltene Schiff'sche Base kann direkt für die Bindung von Antikörpern verwendet werden. Diese können, wenn sie mit Ferritin, Meerrettich-Peroxidase oder ¹²⁵J markiert sind, direkt bestimmt werden. Die gebundenen Antikörper können aber auch nach der sogenannten "Sandwich"-Methode durch die Bindung gegen sie gerichteter zusätzlicher Antikörper bestimmt werden.

Die durch Bindung des 3-(2-Aminoethylthio)propylglycosids erhaltene Schiff'sche Base kann durch Reaktion mit Natriumcyanoborhydrid in ein sekundäres Amin umgewandelt werden. 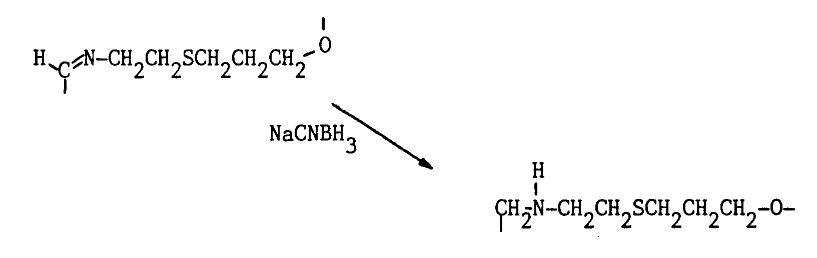
Die Bindung ist dann gegen Säuren stabiler.

Die Bestimmung der freien Aldehydgruppen im Polysaccharidanteil nach der Oxidation mit Perjodat erfolgt entsprechend der gängigen Methoden mit Phenylhydrazin oder 2,4-Dinitrophenylhydrazin.

Geeignete kohlenhydrathältige S-Schichten werden wie in Beispiel 1 unter A und B beschrieben, mit Natriummetaperjodat oxidiert. Jodat und Perjodat werden durch Dialyse gegen Wasser entfernt. Dann wird eine Lösung des entsprechenden Hydrazinderivates in 10%-iger Essigsäure zugefügt und das ganze 1 Stunde reagieren gelassen.

Dann wird das überschüssige Reagens durch Dialyse entfernt und die Menge der gebildeten Hydrazongruppen kolorimetrisch gemessen.

Die Methode kann auch zur Bestimmung der freien Restgruppen nach der Bindung eines aminogruppenhaltigen Haptens und/oder der immunogenen bzw. immunstimulierenden Substanzen eingesetzt werden. 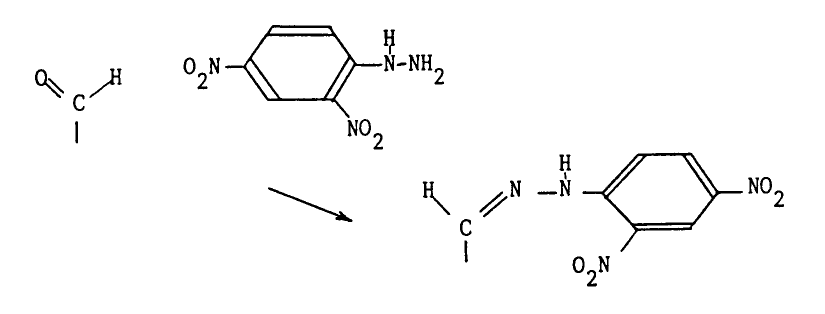
Die erfindungsgemäßen pharmazeutischen Strukturen eignen sich besonders als Immunisierungsantigene zur Erzielung hoher Antikörpertiter. Im Falle der Verwendung von Antikörpern als immunogene Substanzen lassen sich solcherart antiidiotypische Antikörper erhalten. Weiters sind die Strukturen vorteilhaft als kombiniertes System Immunisierungsantigen-Booster (ein und dasselbe Hapten und/oder immunogene bzw. immunstimulierende Substanz, aufgepropft auf zwei aus verschiedenen Stämmen stammende S-Schicht-Glycoprotein bzw. Protein-Spezies). Die Strukturen sind auch als Immunsorbentien, z.B. für diagnostische Kits oder extrakorporeale Depletion unerwünschter Antikörper (Blutwäsche) anwendbar.

## Patentansprüche

1. Pharmazeutische Struktur, in welcher Haptene und/oder immunogene bzw. immunstimulierende Substanzen an einem Proteinträger gebunden sind, dadurch gekennzeichnet, daß der Proteinträger durch die kristallin oder parakristallin aneinanderliegenden, gegebenenfalls miteinander kovalent vernetzten, Proteinmoleküle oder proteinhältigen Moleküle einer S-Schicht gebildet ist.

2. Pharmazeutische Struktur nach Anspruch 1, dadurch gekennzeichnet, daß die kristallin oder parakristallin aneinanderliegenden proteinhältigen Moleküle Glycoproteine sind.

3. Pharmazeutische Struktur nach Anspruch 1 oder 2, dadurch gekennzeichnet, daß die kristallin aneinanderliegenden Moleküle aus einer oder mehreren Mikroorganismenzellwandschichten stammen.

4. Pharmazeutische Struktur nach einem der Ansprüche 1 bis 3, dadurch gekennzeichnet, daß an dem kristallinen oder parakristallinen Proteinträger zusätzlich weitere Zellwandbestandteile anhaften.

5. Pharmazeutische Struktur nach Anspruch 3 oder 4, dadurch gekennzeichnet, daß Fragmente von Mikroorganismenzellwänden mit den Haptenen und/oder immunogenen bzw. immunstimulierenden Substanzen beladen sind.

6. Pharmazeutische Struktur nach einem der Ansprüche 1 bis 5, dadurch gekennzeichnet, daß die Haptene und/oder immunogenen bzw. immunstimulierenden Substanzen an den Proteinanteil des Trägers gebunden sind.

7. Pharmazeutische Struktur nach einem der Ansprüche 2 bis 5, dadurch gekennzeichnet, daß die Haptene und/oder immunogenen bzw. immunstimulierenden Substanzen an den Kohlenhydratanteil der Glycoproteine gebunden sind.

8. Pharmazeutische Struktur nach Anspruch 6 oder 7, dadurch gekennzeichnet, daß die Haptene und/oder immunogenen bzw. immunstimulierenden Substanzen an dem jeweiligen Trägermolekül über Zwischenmoleküle, wie z.B. homo- oder heterobifunktionelle quervernetzende Agentien (Crosslinker) oder Peptidketten (z.B. Polylysin) gebunden sind.

9. Pharmazeutische Struktur nach einem der Ansprüche 1 bis 8, dadurch gekennzeichnet, daß unterschiedliche Träger und/oder mit unterschiedlichen Haptenen und/oder immunogenen bzw. immunstimulierenden Substanzen beladene Träger vereinigt sind.

10. Verfahren zur Herstellung der pharmazeutischen Struktur nach einem der Ansprüche 1 bis 9, dadurch gekennzeichnet, daß die die Haptene und/oder immunogenen bzw. immunstimulierenden Substanzen bindenden Gruppen der Proteinmoleküle oder proteinhältigen Moleküle vor dem Zusatz der Haptene und/oder immunogenen bzw. immunstimulierenden Substanzen aktiviert werden.

11. Verfahren nach Anspruch 10 und 7, dadurch gekennzeichnet, daß Bindungsstellen an den Kohlenhydratanteilen der Glycoproteine durch Oxidation, beispielsweise mit Perjodat, geschaffen werden.

12. Verfahren nach Anspruch 10 und 6, dadurch gekennzeichnet, daß die Bindung von Haptenen und/oder immunogenen bzw. immunstimulierenden Substanzen an den Proteinmolekülen durch das die Proteinmoleküle kovalent vernetzende Agens, z.B. Glutaraldehyd, bewirkt wird.

13. Verfahren nach Anspruch 12, dadurch gekennzeichnet, daß die Bildung der Bindungsstellen durch Einführung aktiver Gruppen vorgenommen wird.

14. Verfahren nach Anspruch 10, dadurch gekennzeichnet, daß die Haptene und/oder immunogenen bzw. immunstimulierenden Substanzen an die Carboxylgruppen des Proteinträgers amidartig gebunden werden.

15. Verfahren nach Anspruch 11, dadurch gekennzeichnet, daß die Bindung der Haptene und/oder immunogenen bzw. immunstimulierenden Substanzen in Form von Schiff'schen Basen erfolgt, wobei gegebenenfalls die Schiff'schen Basen zu sekundären Aminen reduziert werden.

16. Verfahren zur Bildung einer pharmazeutischen Struktur nach einem der Ansprüche 1 bis 9, dadurch gekennzeichnet, daß die Bindungsstellen am Hapten und/oder an der immunogenen bzw. immunstimulierenden Substanz aktiviert werden, und das Hapten und/oder die immunogene bzw. immunstimulierende Substanz über diese aktivierten Bindungsstellen an NH₂-Gruppen des Proteinträgers gebunden wird.

17. Verfahren zur Bildung einer pharmazeutischen Struktur nach Anspruch 8, dadurch gekennzeichnet, daß an beiden Enden aktivierte Bindungsstellen aufweisende Zwischenmoleküle, wie z.B. homo- oder heterobifunktionelle quervernetzende Agentien (Crosslinker) oder Peptidketten (z.B. Polylysin), mit einem Ende an den Träger gebunden werden, wonach dann das Hapten und/oder die immunogene bzw. immunstimulierende Substanz an das andere Ende des Zwischenmoleküls gebunden wird.

18. Verfahren zur Bildung einer pharmazeutischen Struktur nach Anspruch 9, dadurch gekennzeichnet, daß unterschiedliche Träger und/oder mit unterschiedlichen Haptenen und/oder immunogenen bzw. immunstimulierenden Substanzen beladene Träger auf eine Hilfsschicht aufgebracht werden, welche nach Vernetzen der Träger gegebenenfalls entfernt wird.

## Claims

1. Pharmaceutical structure in which haptens and/or immunogenic or immunostimulating substances are bonded to a protein carrier, characterised in that the protein carrier is formed by the crystalline or paracrystalline contiguous protein molecules or protein-containing molecules, which are optionally covalently cross-linked with one another, of an S-layer.

2. Pharmaceutical structure as claimed in claim 1, characterised in that the crystalline or paracrystalline contiguous protein-containing molecules are glycoproteins.

3. Pharmaceutical structure as claimed in claim 1 or 2, characterised in that the crystalline contiguous molecules originate from one or a plurality of microorganism cell wall layers.

4. Pharmaceutical structure as claimed in one of claims 1 to 3, characterised in that further cell wall components additionally adhere to the crystalline or paracrystalline protein carrier.

5. Pharmaceutical structure as claimed in claim 3 or 4, characterised in that fragments of microorganism cell walls are loaded with the haptens and/or immunogenic or immunostimulating substances.

6. Pharmaceutical structure as claimed in one of claims 1 to 5, characterised in that the haptens and/or immunogenic or immunostimulating substances are bonded to the protein component of the carrier.

7. Pharmaceutical structure as claimed in one of claims 2 to 5, characterised in that the haptens and/or immunogenic or immunostimulating substances are bonded to the carbohydrate component of the glycoproteins.

8. Pharmaceutical structure as claimed in claim 6 or 7, characterised in that the haptens and/or immunogenic or immunostimulating substances are bonded to the respective carrier molecule via intermediate molecules, such as homo- or heterobifunctional cross-linking agents (Crosslinkers) or peptide chains (e.g. polylysine).

9. Pharmaceutical structure as claimed in one of claims 1 to 8, characterised in that different carriers and/or carriers loaded with different haptens and/or immunogenic or immunostimulating substances are combined.

10. Method of manufacturing the pharmaceutical structure as claimed in one of claims 1 to 9, characterised in that the groups of the protein molecules or protein-containing molecules which bond the haptens and/or immunogenic or immunostimulating substances are activated before the addition of the haptens and/or immunogenic or immunostimulating substances.

11. Method as claimed in claims 10 and 7, characterised in that bonding sites are produced on the carbohydrate components of the glycoproteins by oxidation, for instance with periodate.

12. Method as claimed in claims 10 and 6, characterised in that the bonding of haptens and/or immunogenic or immunostimulating substances to the protein molecules is effected by the agent which covalently cross-links the protein molecules, e.g. gluteraldehyde.

13. Method as claimed in claim 12, characterised in that the formation of the bonding sites is effected by introducing active groups.

14. Method as claimed in claim 10, characterised in that the haptens and/or immunogenic or immunostimulating substances are bonded via an amide linkage to the carboxyl groups of the protein carrier.

15. Method as claimed in claim 11, characterised in that the bonding of the haptens and/or immunogenic or immunostimulating substances occurs in the form of Schiff bases, the Schiff bases being optionally reduced to secondary amines.

16. Method of forming a pharmaceutical structure as claimed in one of claims 1 to 9, characterised in that the bonding sites to the hapten and/or to the immunogenic or immunostimulating substance are activated and the hapten and/or the immunogenic or immunostimulating substance is bonded via these activated bonding sites to NH₂ groups of the protein carrier.

17. Method of forming a pharmaceutical structure as claimed in claim 8, characterised in that intermediate molecules having activated bonding sites at both ends, such as homo- or heterobifunctional cross-linking agents (Crosslinkers) or peptide chains (e.g. polylysine) are bonded with one end to the carrier, whereafter the hapten and/or the immunogenic or immunostimulating substance is bonded to the other end of the intermediate molecule.

18. Method of forming a pharmaceutical structure as claimed in claim 9, characterised in that different carriers and/or carriers loaded with different haptens and/or immunogenic or immunostimulating substances are applied to an auxiliary layer which is optionally removed after cross-linking of the carrier.

## Revendications

1. Complexe pharmaceutique dans lequel des haptènes et/ou des substances immunogènes et/ou inductrices d'une immuno-réactivité sont liées à un porteur protéique, caractérisé en ce que le porteur protéique est constitué par des molécules protéiques ou des molécules comprenant des protéines, d'une couche en S, lesdites molécules ayant une structure cristalline ou paracristalline, adjacentes et étant, le cas échéant, réticulées par des liaisons covalentes.

2. Complexe pharmaceutique selon la revendication 1, caractérisé en ce que les molécules comprenant des protéines et ayant une structure cristalline ou paracristalline, adjacentes, sont des glycoprotéines.

3. Complexe pharmaceutique selon la revendication 1 ou 2, caractérisé en ce que les molécules de structure cristalline adjacentes sont issues d'une ou de plusieurs couches de la paroi cellulaire de micro-organismes.

4. Complexe pharmaceutique selon l'une des revendications 1 à 3, caractérisé en ce que d'autres composants de la paroi cellulaire adhèrent au porteur protéique de structure cristalline ou paracristalline.

5. Complexe pharmaceutique selon la revendication 3 ou 4, caractérisé en ce que des fragments de la paroi cellulaire de micro-organismes portent des haptènes et/ou des substances immunogènes et/ou inductrices d'immuno-réactivité.

6. Complexe pharmaceutique selon l'une des revendications 1 à 5, caractérisé en ce que les haptènes et/ou les substances immunogènes et/ou inductrices d'immuno-réactivité sont liés à la partie protéique du porteur.

7. Complexe pharmaceutique selon l'une des revendications 2 à 5, caractérisé en ce que les haptènes et/ou les substances immunogènes et/ou inductrices d'immuno-réactivité sont liés à la partie glucidique de la glycoprotéine.

8. Complexe pharmaceutique selon la revendication 6 ou 7, caractérisé en ce que les haptènes et/ou les substances immunogènes et/ou inductrices d'immuno-réactivité sont liés à leur molécule porteuse grâce à des molécules de couplage, comme par exemple des agents de réticulation (crosslinker) homo- ou hétérobifonctionnalisés, ou des chaînes peptidiques (par exemple polylysine).

9. Complexe pharmaceutique selon l'une des revendications 1 à 8, caractérisé en ce que différents porteurs sont combinés avec divers porteurs liés à des haptènes et/ou des substances immunogènes et/ou inductrices d'immuno-réactivité.

10. Procédé de fabrication du complexe pharmaceutique selon l'une des revendications 1 à 9, caractérisé en ce que les groupements des molécules protéiques ou des molécules comprenant des protéines qui sont liées aux haptènes et/ou aux substances immunogènes et/ou inductrices d'immuno-réactivité sont activés avant l'addition des haptènes et/ou des substances immunogènes et/ou inductrices d'immuno-réactivité.

11. Procédé selon la revendication 10 et 7, caractérisé en ce que des sites de liaison sont créés sur la partie glucidique de la glycoprotéine par oxydation, par exemple à l'aide de périodate.

12. Procédé selon la revendication 10 et 6, caractérisé en ce que la liaison créée entre les haptènes et/ou les substances immunogènes et/ou inductrices d'immuno-réactivité et les molécules protéiques est réalisée au moyen de l'agent qui réticule de manière covalente la molécule protéique, par exemple le glutaraldéhyde.

13. Procédé selon la revendication 12, caractérisé en ce que la formation de sites de liaison est réalisée en introduisant des groupements activés.

14. Procédé selon la revendication 10, caractérisé en ce que les haptènes et/ou les substances immunogènes et/ou inductrices d'immuno-réactivité sont liés au groupement carboxylique du porteur protéique pour former une fonction amide.

15. Procédé selon la revendication 11, caractérisé en ce que la liaison des haptènes et/ou des substances immunogènes et/ou inductrices d'immuno-réactivité se présente sous la forme d'une base de Schiff, les bases de Schiff étant, le cas échéant, réduites sous forme d'une amine secondaire.

16. Procédé de fabrication d'un complexe pharmaceutique selon l'une des revendications 1 à 9, caractérisé en ce que les sites de liaison des haptènes et/ou des substances immunogènes et/ou inductrices d'immuno-réactivité sont activés, et en ce que l'haptène et/ou la substance immunogène et/ou inductrice d'immuno-réactivité est lié à des groupes NH₂ du porteur protéique par l'intermédiaire de ces sites de liaison activés.

17. Procédé de fabrication d'un complexe pharmaceutique selon la revendication 8, caractérisé en ce que les molécules intermédiaires présentant des sites de liaison, activées aux deux extrémités, comme par exemple les agents de réticulation homo- ou hétérobifonctionnalisés (crosslinkers) ou les chaînes peptidiques (par exemple polylysine), sont liées au porteur à une de leurs extrémités, et à l'haptène et/ou à la substance immunogène et/ou inductrice d'immunoréactivité à leur autre extrémité.

18. Procédé de fabrication d'une strucutre pharmaceutique selon la revendication 9, caractérisé en ce que différents porteurs et/ou divers porteurs liés à des haptènes et/ou des substances immunogènes et/ou inductrices d'immuno-réactivité sont disposés sur une couche auxiliaire, laquelle peut éventuellement être retirée après la réticulation du porteur.
